# EUROPEAN PATENT APPLICATION

(11) **EP 3 400 954 A1**
(43) Date of publication of application: **14.11.2018**
(21) Application number: 17305527.8
(22) Date of filing: 09.05.2017
(51) Int. Cl.: A61K 38/08, A61K 38/45, A61K 39/00, A61P 35/00, C07K 7/06

(54) **VACCINE TARGETING A CRYPTIC TERT EPITOPE, FOR TREATING LUNG CANCER IN A HLA-A*0201-POSITIVE NEVER-SMOKER OR LIGHT-FORMER SMOKER PATIENT**

(71) Applicant: Vaxon Biotech, 75749 Paris Cedex 15 (FR)
(72) Inventor: KOSMATOPOULOS, Kostantinos (Kostas), 75005 PARIS (FR); MENEZ-JAMET, Jeanne, 92120 MONTROUGE (FR)
(74) Representative: Santarelli

(57) **Abstract**

The invention pertains to the use of a tumor vaccine composed of two peptides of nine amino acids - the WT cryptic TERT572 (RLFFYRKSV, SEQ ID No: 1) expressed by tumor cells and its optimized variant TERT572Y (YLFFYRKSV, SEQ ID No: 2) - for treating cancer in a HLA-A*0201-positive patient having a non small cell lung cancer (NSCLC) expressing TElomerase Reverse Transcriptase (TERT), wherein said patient is a never-smoker or a light-former smoker.

## Description

### FIELD OF THE INVENTION

The present invention pertains to the field of cancer immunotherapy, and more particularly to the field of antitumor vaccination.

### BACKGROUND AND PRIOR ART

Cancer immunotherapy is intended to stimulate cytotoxic T lymphocytes (CTL) recognizing peptides derived from tumor antigens and presented at the tumor cell surface by HLA class I molecules. CTL-targeted peptides can be dominant or cryptic depending on their affinity for MHC molecules.

Tumor-associated antigens (TAA) are frequently expressed by both tumor cell and normal tissues, contrary to neoantigens that are tumor-specific and most often patient-specific. To circumvent the problem of tolerance to TAA while still targeting an antigen widely expressed by tumors, the inventors proposed a vaccine (Vx-001) targeting a cryptic peptide from TElomerase Reverse Transcriptase (TERT), *i.e.*, a peptide that exhibits a low affinity for the HLA-A*0201 molecule and forms unstable peptide/HLA-I complexes (Jeanne Menez-Jamet *et al.*, 2016). Given the strong correlation between HLA-I affinity and immunogenicity, this cryptic peptide is naturally not immunogenic. To use it as a cancer vaccine, this cryptic peptide was hence optimized to enhance its immunogenicity.

Vx-001 is thus composed of two peptides of nine amino acids: the WT cryptic TERT572 (RLFFYRKSV, SEQ ID No: 1) expressed by tumor cells and its optimized variant TERT572Y (YLFFYRKSV, SEQ ID No: 2). These two peptides are administered separately, along with the adjuvant Montanide ISA51®VG (a mixture of a highly purified mineral oil (Drakeol 6VR) and a surfactant (Mannide monooleate)). The optimized immunogenic TERT572Y is given in the first two vaccinations, in order to trigger a large immune response. The WT TERT572 is given in the following vaccinations, in order to select among all the TERT572Y stimulated T cells those with the highest specificity for the WT TERT572 that is presented on the surface of tumor cells associated with the HLA-A*0201.

Non small cell lung cancer (NSCLC), including adenocarcinoma, squamous cell carcinoma, and large cell carcinoma, is the most common type of lung cancer, representing about 80% of lung cancer cases; it accounts for approximately 1.2 million new cases annually worldwide.

The clear majority (85%) of cases of lung cancer are due to long-term tobacco smoking. The association of smoking as a risk factor for lung cancer has been established since the 1950s.

Nevertheless, measuring the tobacco exposure for one individual is not obvious since all smokers do not smoke the same amount of cigarettes, and smokers often vary their smoking habits over the years. In addition, there is a degree of individual variability in susceptibility to tobacco mutagens. Several methods were thus proposed to quantify smoking-associated risk factor to stratify cancer patients according to objective parameters.

A first method consists in measuring the amount a person has smoked over a long period of time, using the "pack-year" unit. A pack year is defined as twenty cigarettes smoked every day for one year. The number of pack-years for one individual is calculated by multiplying the number of packs of cigarettes smoked per day by the number of years the person has smoked this amount of cigarettes each day. For example, 1 pack-year is equal to smoking 20 cigarettes (1 pack) per day for 1 year, or 40 cigarettes per day for half a year, or 10 cigarettes per day during two years, *etc.* Hence, this parameter takes into account not only the duration of smoking, but also its intensity.

Other methods are based on the identification of smoking molecular signatures, which reflect the consequences of smoking in each individual. In a recent study, Rizvi *et al.* demonstrated the existence of a molecular smoking signature based on the amount of transversion mutations in tumor cells (Rizvi *et al.* 2015). This parameter focuses on the effects of smoking rather than on the amount of tobacco smoked by an individual, and distinguishes transversion-high (TH, smoking signature) from transversion-low (TL, never-smoking signature) tumors.

Another molecular smoking signature, based on the amount and/or nature of mutations in *EGFR* and/or *KRAS* genes, was described by Dogan *et al.* (2012). In this publication, the authors reported that in lung cancers, *EGFR* mutations were far more frequently present in tumors from never-smokers than in those from smokers, contrary to mutations in *KRAS,* which occurred more frequently in smokers. They observed that independently of pack-years, increasing smoking-free years raise the likelihood of *EGFR* mutations. They also showed that the most frequent G>T transversion mutation in smokers (*KRAS* G12C) was more frequent in women and that these women were younger than men with the same mutation, suggesting that women are more susceptible than men to tobacco carcinogens.

About 10-15% of lung cancer cases occur in people who have never or lightly smoked. Lung cancer not related to smoking may be due to other risk factors including environment, hormones, menstrual cycle and viral infection.

Importantly, lung cancer not related to smoking has a worse prognostic than lung cancer related to smoking.

Smoking is highly related to high tumor mutations burden, whereas lung cancer not related to smoking is characterized by a low level of tumor mutations (Rizvi, *et al.* 2015). That is why lung cancer in never- or light-smokers is not sensitive to immune check-point inhibitors, contrary to smoking-induced lung cancer.

Vx-001 was tested in a randomized phase IIb clinical trial in metastatic or recurrent stage I-III NSCLC patients who experienced disease control after four cycles of platinum-based chemotherapy.

Patients needed to be HLA-A*0201 positive with a tumor expressing TERT. The primary objective of the study was overall survival. The result of this study was globally not statistically significant, but a detailed analysis of the results, taking into account analysis the smoking history of the patients, as well other characteristics of the patients such as their specific immune response to the vaccine (immunomonitoring data), their age *etc.*, led to a stratification of the patients with identification of categories of patients for whom vaccination by Vx-001 proved beneficial.

### SUMMARY OF THE INVENTION

The invention relates to the selection of patients more likely to respond to vaccination by Vx-001, a vaccine composed of two peptides (SEQ ID No:1 and SEQ ID No: 2) administered separately.

The invention pertains to the use of this vaccine and of each of its components, for treating cancer in a HLA-A*0201-positive patient having a non small cell lung cancer (NSCLC) expressing TElomerase Reverse Transcriptase (TERT), wherein said patient is either a never-smoker or a light-smoker, *i.e.*, has been a smoker during less than 25 years or less than 30 years. In case the patient is a light-smoker, the patient is preferably a light-former smoker, *i.e.*, a light-smoker who stopped smoking before NSCLC diagnosis.

The invention also pertains to a theranostic method to determine whether a HLA-A*0201-positive never-smoker or light-smoker patient having a NSCLC expressing TERT is more likely to respond to Vx-001, wherein the patient is more likely to be a good responder if the tumor is non-immunogenic.

Another aspect of the present invention is a kit to perform the theranostic method.

### LEGENDS TO THE FIGURES

Figure 1: Vaccination protocol
Figure 2: Response to different tumor antigens of patients with (left) or without (right) natural immunity
Figure 3: Clinical outcome (OS and TTF) of all evaluated patients
Figure 4: Clinical outcome of never-smokers and light former smokers (who have been smoking during less than 25 years) 29 placebo and 26 vaccinated; 11.2 vs 16.2 mo; p=0.07; HR=0.59 (0.32 - 1.06)
Figure 5: Clinical outcome of never-smokers and light former smokers (who have been smoking during less than 25 years) without natural immunity. 16 placebo and 17 vaccinated; 7.9 vs 20.7 mo; p=0.0007; HR=0.29 (0.13 - 0.67)
Figure 6: Clinical outcome of never-smokers and light former smokers (who have been smoking during less than 25 years) who entered the study with a stable disease (SD) after a first-line chemotherapy. 14 placebo and 15 vaccinated. 12.4 vs 24.7 m; p=0.004; HR=0.33 (0.14 - 0.78)
Figure 7: Clinical outcome of male never-smokers and light former smokers (who have been smoking during less than 25 years). 18 placebo and 14 vaccinated. 11.1 vs 24.7 m. p=0.01; HR=0.37 (0.16 - 0.81)

### DETAILED DESCRIPTION OF THE PREFERRED EMBODIMENTS

According to a first embodiment, the present invention pertains to the use of the optimized peptide TERT572Y (peptide of SEQ ID No: 2), for inducing an anti-tumor CTL response in a HLA-A*0201-positive patient having a NSCLC expressing TERT, wherein said patient is either a never-smoker or a light-smoker.

When performing this anticancer immunotherapy, vaccination with TERT572Y induces a CTL response against the cryptic peptide TERT572 (SEQ ID No: 1).

As used herein, the terms "treat", "treatment" and "treating" refer to any delay of the progression, reduction of severity such as amelioration of quality of life thereof and/or an increase in survival that results from the administration of one or more therapies.

The present invention also pertains to the use of the native peptide TERT572 (peptide of SEQ ID No: 1), for treating lung cancer in a HLA-A*0201-positive patient having a NSCLC expressing TERT, wherein said patient is either a never-smoker or a light-smoker. In the frame of this immunotherapy treatment, the peptide of SEQ ID No: 1 maintains a CTL immune response initiated by vaccination of the patient with a peptide of SEQ ID No: 2.

The present invention also relates to the use of Vx-001, *i.e.* the combination of peptides of SEQ ID No: 1 and SEQ ID No: 2, in the treatment of lung cancer in a HLA-A*0201-positive patient having a NSCLC expressing TERT, wherein said patient is either a never-smoker or a light-smoker. As already described, the two peptides of Vx-001 are administered separately. A CTL response against the tumor antigen TERT, more precisely against the cryptic TERT572 peptide of SEQ ID No: 1, is first induced by vaccinating the patient with the peptide of SEQ ID No: 2 and then maintained by vaccinating the patient with the peptide of SEQ ID No: 1.

As used herein, a "smoker" designates an individual who smokes (current smoker) or does not smoke anymore but has been smoking earlier in his life (former smoker), whatever the amount of tobacco smoked each day/week/month; the duration of smoking can correspond to one single period in the individual's life or to the addition of several periods if the individual interrupted his/her smoking habits.

A "never-smoker" is an individual who has smoked less than 20 cigarettes in his/her lifetime.

In the experimental part below, a "light-smoker" is an individual who has been a smoker during at most 30 years, preferably at most 25 years. This parameter has been defined as such herein for two reasons: (i) the only available information regarding the smoking history of the patients in the cohort of the phase IIb study was the number of years during which they regularly smoked; and (ii) this parameter led to the stratification of patients with two populations (never-smokers + former light-smokers on the one hand and heavy-former smokers and current smokers on the other hand) whose responses to Vx-001-vaccination were significantly different. Of course, this definition of "light-smokers" can be changed to use any other parameter for measuring tobacco exposure. Using results of another cohort, the skilled artisan can adapt the definition of "light-smokers" to such an alternative way of measuring tobacco exposure. Non-limitative examples of measuring tobacco exposure are:
- smoking pack-years: as mentioned above, this parameter takes into account not only the duration of smoking, but also its intensity. Of course, the skilled artisan can determine, on a cohort of NSCLC patients, an appropriate pack-years threshold to differentiate light-smokers from heavy-smokers in the frame of the present invention.
- molecular smoking signature based on the amount of transversion mutations in tumor cells: as mentioned above, Rizvi *et al.* (2015) distinguished transversion-high (TH, smoking signature) from transversion-low (TL, never-smoking signature) tumors. Of course, the threshold to differentiate the populations (amount of transversions observed in the tumor) can be adapted to include some smokers (with an intermediate number of transversion mutations) into a light-smoker category with never-smokers and determine a heavy-smoking signature for patients exhibiting more transversions. This way of discriminating light- from heavy-smokers is advantageous because it is independent from patients' declarations (which can be biased) and from individual susceptibility to tobacco-induced mutations.
- molecular smoking signature based on the amount and/or nature of mutations in *EGFR* and/or *KRAS* genes: as mentioned above, Dogan *et al.* (2012) described that a molecular smoking signature based on the analysis of certain genes, such as *EGFR* and *KRAS,* can be defined to segregate NSCLC patients who have mutations frequently caused by tobacco from those who have mutations independent from tobacco (never-smoking + light-smoking). Such a molecular signature can of course be refined by the skilled artisan and adapted to discriminate NSCLC patients to identify those most likely to respond to Vx-001 vaccination, according to the present invention.

According to a preferred embodiment of the present invention, Vx-001 is administered to a patient who is a never-smoker or a former smoker who has been a smoker during less than 25 years (or at most 25 years).

According to another particular embodiment, the patient is a never-smoker or a former smoker who stopped smoking at least 5, at least 7, at least 10 or at least 15 years before NSCLC diagnosis.

According to a preferred embodiment illustrated in the experimental part below, the patient first receives 2 vaccinations with the peptide of SEQ ID No: 2 and then 4 vaccinations with the peptide of SEQ ID No:1, with a three week interval between said vaccinations.

According to another embodiment, after the first six vaccinations mentioned above, the patient receives additional vaccinations with the peptide of SEQ ID No:1, to maintain the CTL response against TERT. These additional vaccinations can for example be administered every 3 months. They can be administered until relapse.

According to another particular embodiment of the invention, the patient has received a first line chemotherapy, for example a platinum-based chemotherapy, such as a cisplatin-based chemotherapy before the first vaccination with the peptide of SEQ ID No: 2. In this case, the patient preferably has a non-progressing disease, more preferably a stable disease upon vaccination with the peptide of SEQ ID No: 2.

As illustrated in the experimental part below, the inventors measured CTL responses against a number of tumor antigens before Vx-001 vaccination. By doing so, they showed that patients having a CTL response against TERT572 before the beginning of the immunotherapy with Vx-001 also have CTL responses against other tumor epitopes. Therefore, they hypothesized that some patients have immunogenic tumors, while others (with no significant CTL response against tumor antigens) have non-immunogenic tumors. They also showed that never- and light-former smokers who have a non-immunogenic tumor respond significantly better to Vx-001 than never- and light-former smokers who have an immunogenic tumor. Hence, a preferred embodiment of the present invention is the use of Vx-001 or of its components in the treatment of NSCLC in a HLA-A*0201-positive never-smoker or light-former smoker patient having a non-immunogenic NSCLC expressing TERT.

In the present text, an "immunogenic tumor" designates a tumor that elicits a significant CTL response against tumor antigens. When performing the present invention, the immunogenicity of the tumor can be assessed by measuring, in a blood sample from the patient, the number of CTLs specific for TERT572 (SEQ ID No: 1) and/or the number of CTLs specific for TERT540 (SEQ ID No: 3), and/or the number of CTLs specific for another tumor antigen. For example, the immunogenicity of the tumor can be assessed by measuring the CTL response against Survivin, which is a universal tumor antigen expressed by a large majority of tumors (Andersen and Thor, 2002). In HLA-A*0201-positive patients considered herein, this response can be detected by measuring, in a blood sample from the patient, the number of CTLs specific for the epitope Survivin96 (SEQ ID No: 5).

*A contrario,* the tumor will be considered as "non-immunogenic" if no CTL response specific for TERT572 (SEQ ID No: 1), TERT540 (SEQ ID No: 3) and/or Survivin96 (SEQ ID No: 5) is detected in a blood sample from the patient. The IFNg ELISpot assay described in the experimental part below can be used to detect T cells specific for the TERT572 peptide or for any other tumor epitope. Of course, as described in the experimental part below, in absence of a significant difference between the response to the tumor antigen tested peptide and the response to an irrelevant peptide, it will be considered that there is no response specific for the tested tumor peptide, *i.e.*, that the tumor is non-immunogenic.

Alternatively, the immunogenicity of the tumor can be assessed by measuring the level of tumor-infiltrating lymphocytes (TILs) in a biopsy from the tumor such as with immunoscore (Pages et al., 2009), or determining the profile of genes expression of the tumor using gene profiling methods (Galon J *at al.*, 2013, Rizvi NA et *al*., 2015, Wang, E *et al.* 2013).

The inventors found that in some patients, a CTL response against the tumor is detected after chemotherapy, probably because tumor cell lysis following chemotherapy leads to the release of a large amount of tumor epitopes able to induce an antitumor immune response in these patients. These tumor specific CTLs are highly detectable just after the end of chemotherapy, and the amount of CTLs then decreases in the following weeks. The inventors' hypothesis is that this CTL response appears in patients having an immunogenic tumor, who are those who are fully responsive to other immunotherapies such as anti-PD(L)1 treatment. Therefore, for patients who received a chemotherapy treatment such as a platinum-based first line chemotherapy, the immunogenicity status of the tumor will preferably be assessed less than 2 weeks after the end of said chemotherapy, for example less than 7 days after the end of the chemotherapy.

According to a particular embodiment of the present invention, the tumor is considered as non-immunogenic if no CTL response specific for TERT572 (SEQ ID No: 1) is detectable in a blood sample from the patient collected less than 2 weeks, preferably less than 7 days after the end of a platinum-based first line chemotherapy.

According to another particular embodiment of the present invention, the tumor is considered as non-immunogenic if no CTL response specific for TERT540 (SEQ ID No: 3) is detectable in a blood sample from the patient collected less than 2 weeks, preferably less than 7 days after the end of a platinum-based first line chemotherapy.

According to another particular embodiment of the present invention, the tumor is considered as non-immunogenic if no CTL response specific for Survivin96 (SEQ ID No: 5) is detectable in a blood sample from the patient collected less than 2 weeks, preferably less than 7 days after the end of a platinum-based first line chemotherapy.

According to another particular embodiment of the present invention, the patient is a male. The results of the clinical trial indeed show that amongst never- and light former-smokers, male patients respond better to the treatment.

According to another aspect, the present invention relates to a method for *in vitro* determining whether a HLA-A*0201-positive never-smoker or light-smoker patient having a TERT-positive NSCLC is more likely to be a good responder to an immunotherapy treatment by vaccination with the peptides of SEQ ID Nos: 1 and 2, comprising assessing the immunogenicity of the tumor, wherein if the tumor is non-immunogenic, the patient is more likely to be a good responder to said immunotherapy treatment.

When performing this method, the immunogenicity of the tumor can be assessed by measuring CTL response specific for the peptide of SEQ ID No: 1 and/or by measuring CTL response specific for the peptide of SEQ ID No: 3 and/or by measuring CTL response specific for the peptide of SEQ ID No: 5 in a blood sample from said individual, wherein if no such CTL response is detectable, the tumor is non-immunogenic.

According to a particular embodiment of the above method, CTL response specific for the peptide of SEQ ID No: 1 or for the peptide of SEQ ID No: 3 or for the peptide of SEQ ID No: 5 or for any other relevant tumor-specific peptide is detected by enzyme-linked immunospot (ELISpot) IFNg assay.

According to another particular embodiment of this method, the immunogenicity of the tumor is assessed less than two weeks after the end of a first-line chemotherapy, preferably less than 7 days after the end of a first-line chemotherapy.

Of course, when performing the theranostic method according to the invention, any other method can be used for assessing the immunogenicity of the tumor. In particular, this can be done by measuring the amount of TILs in a biopsy from the tumor or by gene profiling.

The present invention also pertains to a kit of parts for performing the theranostic method described above, comprising (i) reagents and a plate for performing an ELISpot assay, (ii) a peptide selected amongst SEQ ID No: 1, SEQ ID No: 3 and SEQ ID No: 5 and (iii) an irrelevant peptide as negative control.

Another kit according to the invention comprises materials for detecting a molecular smoking signature in tumor cells. For example, such a kit can comprise a DNA probe for detecting specific mutations in certain genes such as EGFR and KRAS.

Another kit of parts according to the invention comprises the reagents for assessing the immunogenicity of the tumor, as well as materials for detecting a molecular smoking signature in tumor cells.

Other characteristics of the invention will also become apparent in the course of the description which follows of the clinical study and biological assays which have been performed in the framework of the invention and which provide it with the required experimental support, without limiting its scope.

### EXPERIMENTAL RESULTS

### Material and Methods

### Study design and participants

The Vx-001-201 study is a randomized, double blind multicenter study done in 70 centers in France, Germany, Spain, Italy, Greece, Poland, Romania and Czech Republic. Key eligibility criteria were a) non small cell lung cancer (NSCLC), b) stage IV or recurrent stage I-III, c) disease control after platinum based first line chemotherapy according to RECIST 1.1 criteria, d) HLA-A*0201 positivity, e) tumors expressing TElomerase Reverse Transcriptase (TERT), f) ECOG 0 or 1 g) absence of brain metastasis.

The study was conducted according to the Declaration of Helsinki and all applicable regulatory and ethical requirements. The study was approved by the Independent Ethics Committee responsible for each study site in accordance with the local legislation. All patients provided written informed consent.

### Endpoints

a) Principal endpoint: overall survival (OS) measured from randomization
b) Secondary endpoints:
   - Time to Treatment Failure (TTF) measured from randomization
   - OS at 12 months
c) Main exploratory objectives
   - Evaluation of vaccine induced specific immune response
   - Correlation between vaccine specific immune response and clinical response

### Procedures and vaccination protocol

Patients who fulfilled all inclusion criteria were randomized within 8 weeks after the end of first line chemotherapy.

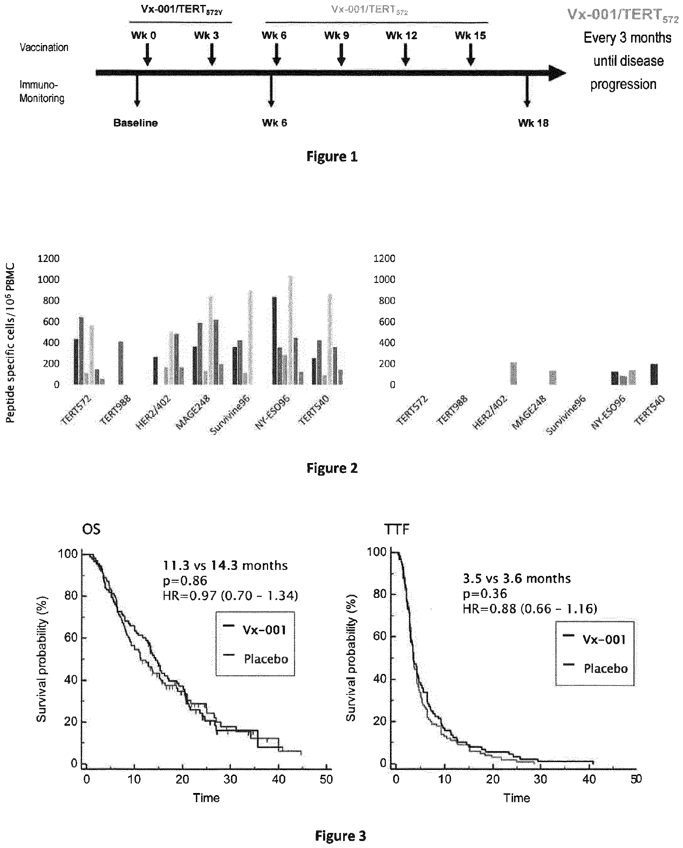

The vaccination protocol consisted in six vaccinations at three week interval. The optimized Vx-001/TERT572Y was used in the first two vaccinations and the native Vx-001/TERT572 in the following four vaccinations. Patients who continued controlling their disease after the sixth vaccination received boost vaccination with the Vx-001/TERT572 every three months. Vaccination was stopped at disease progression (Figure 1).

Immune response was evaluated before the first vaccination (baseline), after the second vaccination (W6) and after the sixth vaccination (W18). Patients who received boost vaccinations were monitored for immune response every six months.

### Statistical analysis

To calculate the sample size we estimated the median OS of the placebo arm to be 9.8 months and we expected the median OS in the Vx-001 arm to be 15.2 months. Thus, we needed to randomize 220 patients (including 10% of drop out at the final analysis) to achieve a power of 83% and a one-side alpha of 0.05.

The placebo : Vx-001 ratio was 1:1.

The primary and secondary endpoints were analysed in the Full Analysis Set composed of all patients who fulfilled the three main criteria a) NSCLC, b) stage IV or recurrent stage I-III and c) disease control after first line chemotherapy.

We used the Kaplan-Meier method to estimate OS and TTF in each arm and a Cox proportional hazards regression model to estimate Hazard Ratio (HR) relating OS and TTF to the treatment effect.

### Immunomonitoring

Immune response was measured using IFNg ELISpot assay to detect T cells specific for the TERT572 peptide. Peripheral Blood Mononuclear Cells (PBMC) were isolated from blood samples collected before vaccination, at W6 (before the third vaccination), W18 (after 6 vaccinations) and every six months thereafter for patients who received boost vaccinations. PBMC were stored at -160°C and tested when study was unblended. 2x10⁵ PBMC/well were overnight stimulated with TERT572 or an irrelevant peptide as negative control or the CEF peptides pool as positive control or phytohemmaglutinin (PHA) as aspecific positive control in plates coated with anti IFNg antibody (Diaclone) in AIMV serum free medium (in 6-plicate). The presence of PBMC was evaluated by measuring the number of spots in presence of PHA. The quality of the PBMC was evaluated by measuring the response to CEF peptides pool (a mixture of polyallelic epitopic peptides from common viruses, flu, HPV and CMV). Samples were considered when they responded to CEF or when all samples from the same patient were unresponsive to CEF (indicating an absence of CEF reactivity due to patient medical history). Number of spots was quantified using a counter and for each conditions the average of the 6 values was calculated. A blood sample was considered responsive to TERT572 or CEF when a) there was a difference higher than 10 spots between the negative control average value and the TERT572 or CEF group average value and b) there was a statistically significant difference between negative control average value and TERT572 or CEF group average value (p<0.05). A patient without pre-vaccination TERT572 response was considered immune responder when a response to TERT572 was detected during the study protocol. Patients with pre-vaccination TERT572 reactivity were considered as immune responders only if (i) this TERT572 reactivity was amplified (at least two folds) after vaccination or if (ii) the patient first lost the pre-vaccination TERT572 reactivity and a new TERT572 reactivity was detected later in the vaccination protocol.

Immune responses to other TERT peptides and to other antigens were also measured using IFNg ELISpot assay. The peptides used to detect T cells are described in Table 1 below.

**Table 1: peptides used to detect specific CTLs**

| Antigen | Peptide | Sequence | SEQ ID No |
|---|---|---|---|
| TERT | TERT 540-548 | ILAKFLHWL | 3 |
| TERT | TERT 988 | DLQVNSLQTV | 4 |
| Survivin | Survivin-3A 96-104 | LTLGEFLKL | 5 |
| NY-ESO-1 | NY-ESO-1 157-165 | SLLMWITQV | 6 |
| HER-2/neu | HER-2/neu402 | TLEEITGYL | 7 |
| MAGE-A | MAGE-A248D9 | YLEYRQVPD | 8 |
| MAGE-A | MAGE-A248G9 | YLEYRQVPG | 9 |

### Results

### Patients

1407 patients were screened and 221 patients were randomized. The main reasons for screening failure were a) patients were HLA-A*0201 negative, b) there was no biopsy material suitable for TERT expression evaluation and c) disease progressed after first line chemotherapy.

Thirty-one patients were excluded from the Full Analysis Set (FAS) analysis because they did not fulfil the main inclusion criteria (25 patients entered the study with progressive disease, 2 patients with a tumor other than NSCLC and 4 patients with no metastatic or recurrent disease). Table 2 shows demographics of the 190 patients of the FAS.

**Table 2: Demographics of evaluated patients. NSQ: non squamous. SQ: squamous. OR: objective response. SD: stable disease ECOG: Eastern Cooperative Oncology Group scale of performance status.**

| | Placebo | | Vx-001 | |
|---|---|---|---|---|
| **All patients** | 101 | | 89 | |

| **Sex** | | | | |
|---|---|---|---|---|
| Male | 72 | 71% | 60 | 67% |
| Female | 29 | 29% | 29 | 33% |

| **Age** | | | | |
|---|---|---|---|---|
| >65 years | 54 | 54% | 45 | 50% |
| <65 years | 47 | 46% | 44 | 50% |

| **Histology** | | | | |
|---|---|---|---|---|
| NSQ | 59 | 59% | 55 | 62% |
| SQ | 38 | 37% | 34 | 38% |
| Mixt | 4 | 4% | 0 | 0% |

| **Response to 1st line chemotherapy** | | | | |
|---|---|---|---|---|
| OR | 52 | 52% | 36 | 40% |
| SD | 49 | 48% | 53 | 60% |

| **ECOG** | | | | |
|---|---|---|---|---|
| 0 | 42 | 42% | 33 | 37% |
| 1 | 59 | 58% | 56 | 63% |

| **Stage** | | | | |
|---|---|---|---|---|
| IV | 89 | 89% | 73 | 82% |
| recurrent | 12 | 11% | 16 | 18% |

| **Smoking status** | | | | |
|---|---|---|---|---|
| Never smoker | 15 | 15% | 10 | 11% |
| Former smoker (all) | 52 | 51% | 63 | 71% |
| Former smoker (≤20 years) | 10 | 10% | 12 | 13% |
| Former smoker (≤25 years) | 14 | 14% | 16 | 18% |
| Former smoker (<30 years) | 17 | 17% | 19 | 21% |
| Former smoker (≥30 years) | 35 | 35% | 44 | 50% |
| Current | 34 | 34% | 16 | 18% |

Patients were randomized within 8 weeks after the end of chemotherapy.

### Immune response in FAS patients

TERT572 specific immune response was detected before vaccination (natural immunity) in 45 out of 166 evaluable patients (27.1 %). Percentage of patients with natural immunity was 24.1% and 30.4% in placebo treated and Vx-001 treated patients respectively.

Natural immunity was not limited to TERT572 but extended to other tumor antigens. Blood samples of baseline of six patients with natural immunity and three patients without natural immunity were tested against six additional antigens overexpressed in NSCLC. All patients with natural immunity to TERT572 had T cells reactive to other tumor antigens (such as TERT988, TERT540, MAGE248, HER402, Survivin96, NY-ESO96) while patients without natural immunity to TERT572 did not respond to other tumor antigen and in the rare case a response was detected, it was very weak (Figure 2).

These results strongly suggest that patient with natural immunity have immunogenic tumors while patients without natural immunity have non-immunogenic or poorly immunogenic tumors.

In Vx-001 treated patients, TERT572 specific immune response was evaluated in 79 patients at baseline, 73 patients at W6, 42 patients at W18 and 16 patients who received boost vaccinations. Overall, immune response was measured at least at W6, W18 or thereafter in 75 patients. Twenty-two patients mounted a TERT572 specific immune response (29.3%). Surprisingly this response was significantly more frequent in non-squamous (NSQ) than in squamous (SQ) NSCLC (36% vs 13.3%, p=0.037).

The frequency of patients with vaccine-induced immune response was not statistically different between patients with and patients without natural immunity (15% vs 36.2% p=0.14) even if vaccine-induced immune response was more frequent in patients without natural immunity.

### Clinical response

Analysis of the FAS patients showed that there was no significant difference in OS between placebo treated and Vx-001 treated patients (11.3 vs 14.3 months, p=0.86, HR=0.97, 95% Cl 0.70-1.34). There was significant difference neither in TTF (3.5 vs 3.6 months, p=0.36, HR=0.88, 95% Cl 0.66-1.16) nor in 12 month survival (49.5% vs 58%, p=0.24) (Figure 3).

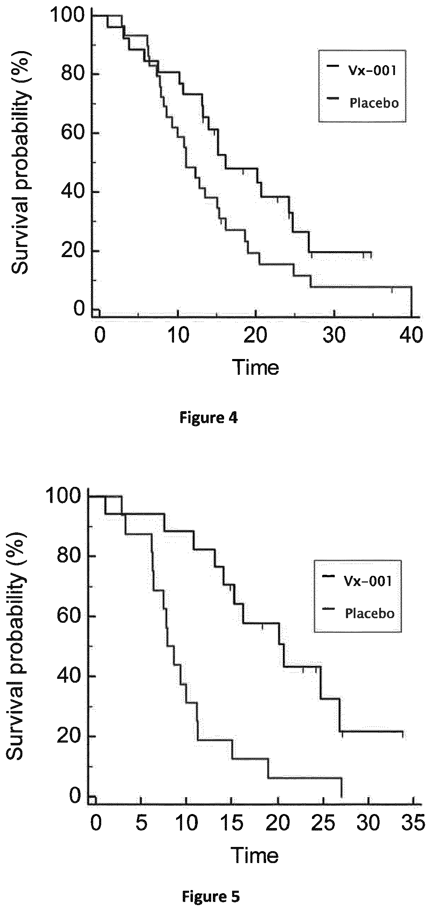

Subgroups analysis showed no significant difference in OS and 12 months survival (table 3) in either subgroup tested excepted the 12 months survival that was significantly higher in males than in females (43% vs 61%, p=0.05). Moreover there was a strong trend to significance in patients who were never smokers or light former smokers (≤25 years) (OS 11.2 vs 16.2, p=0.07) (Figure 4).

**Table 3: Sub-group analysis for OS and 12 months survival**

| Sub-group | Median survival (months) | | P value | HR | 95% CI | 12 months OS (%) | | P value |
|---|---|---|---|---|---|---|---|---|
| | Placebo | Vx-001 | | | | Placebo | Vx-001 | |
| **Histology** | | | | | | | | |
| NSQ (n=118) | 11.3 | 13.4 | 0.56 | 0.88 | 0.58-1.34 | 50 | 55 | 0.58 |
| SQ (n=72) | 11.2 | 14.3 | 0.64 | 1.13 | 0.66-1.93 | 46 | 64 | 0.16 |

| **Response to chemotherapy** | | | | | | | | |
|---|---|---|---|---|---|---|---|---|
| OR (n=87) | 11.1 | 15.3 | 0.64 | 0.89 | 0.54-1.45 | 48 | 69 | 0.07 |
| SD (n=103) | 12.4 | 14.3 | 0.98 | 0.99 | 0.64-1.55 | 51 | 52 | 1 |

| **1st line chemotherapy** | | | | | | | | |
|---|---|---|---|---|---|---|---|---|
| CARBO (n=119) | 12.8 | 14.3 | 0.76 | 1.06 | 0.69-1.63 | 50 | 58.2 | 0.46 |
| CDDP (n=69) | 9.9 | 15 | 0.44 | 0.81 | 0.48-1.32 | 46 | 59 | 0.33 |

| **Age** | | | | | | | | |
|---|---|---|---|---|---|---|---|---|
| >65 years (n=98) | 10 | 15 | 0.29 | 0.78 | 0.50-1.24 | 42.6 | 59 | 0.11 |
| <65 years (n=92) | 15.4 | 13.4 | 0.36 | 1.2 | 0.77-2.00 | 55.3 | 57.7 | 0.83 |

| **Sex** | | | | | | | | |
|---|---|---|---|---|---|---|---|---|
| Males (n=131) | 9.9 | 15 | 0.29 | 0.81 | 0.55-1.20 | 43 | 61 | 0.05 |
| Females (n=59) | 15.8 | 13.2 | 0.16 | 1.52 | 0.82-2.79 | 62 | 53.3 | 0.6 |

| **Smoking status** | | | | | | | | |
|---|---|---|---|---|---|---|---|---|
| Never smoker (NS) (n=25) | 11.4 | 14.1 | 0.52 | 0.76 | 0.32-1.79 | 40 | 70 | 0.22 |
| NS +Former smoker (≤20 years)(n=47) | 11.2 | 15.3 | 0.21 | 0.67 | 0.36-1.26 | 48 | 72.7 | 0.13 |
| NS +Former smoker (≤25 years)(n=55) | 11.2 | 16.2 | 0.07 | 0.59 | 0.32-1.05 | 48.2 | 73 | 0.09 |
| NS + Former smoker (<30 years)(n=61) | 11.2 | 16.2 | 0.17 | 0.67 | 0.38-1.19 | 50 | 69 | 0.19 |
| Current (n=50) | 11.1 | 9 | 0.32 | 1.39 | 0.67-2.87 | 50 | 37.5 | 0.54 |

We then focused the analysis on the population of patients who were never or former light (≤25 years) smokers. This patient population is not likely to have any benefit from the treatment with immune check-point inhibitors (Reck *et al.*, 2016, Zhang *et al.,* 2016). Table 4 shows the demographics of this population. There was no major imbalance between placebo and Vx-001 treated patients.

**Table 4: Demographics of never smokers and former smokers (≤25 years)**

| | **Placebo** | | **Vx001** | |
|---|---|---|---|---|
| **All patients** | 29 | | 26 | |

| **Sex** | | | | |
|---|---|---|---|---|
| Male | 18 | 62% | 14 | 54% |
| Female | 11 | 38% | 12 | 46% |

| **Age** | | | | |
|---|---|---|---|---|
| >65 years | 18 | 62% | 13 | 50% |
| <65 years | 11 | 38% | 13 | 50% |

| **Histology** | | | | |
|---|---|---|---|---|
| NSQ | 21 | 72% | 19 | 73% |
| SQ | 6 | 21% | 7 | 27% |
| Mixt | 2 | 7% | 0 | 0% |

| **Response to 1st line chemotherapy** | | | | |
|---|---|---|---|---|
| OR | 15 | 52% | 11 | 42% |
| SD | 14 | 48% | 15 | 68% |

| **1st line chemotherapy** | | | | |
|---|---|---|---|---|
| CARBO | 20 | 69% | 17 | 65% |
| CDDP | 9 | 31% | 9 | 35% |
| CARBO/CDDP | 0 | 0% | 0 | 0% |

| **Natural immunity** | | | | |
|---|---|---|---|---|
| Yes | 8 | 28% | 6 | 23% |
| No | 16 | 55% | 17 | 65% |
| ND | 5 | 17% | 3 | 12% |

| **Stage** | | | | |
|---|---|---|---|---|
| IV | 24 | 83% | 25 | 96% |
| recurrent | 5 | 17% | 1 | 4% |

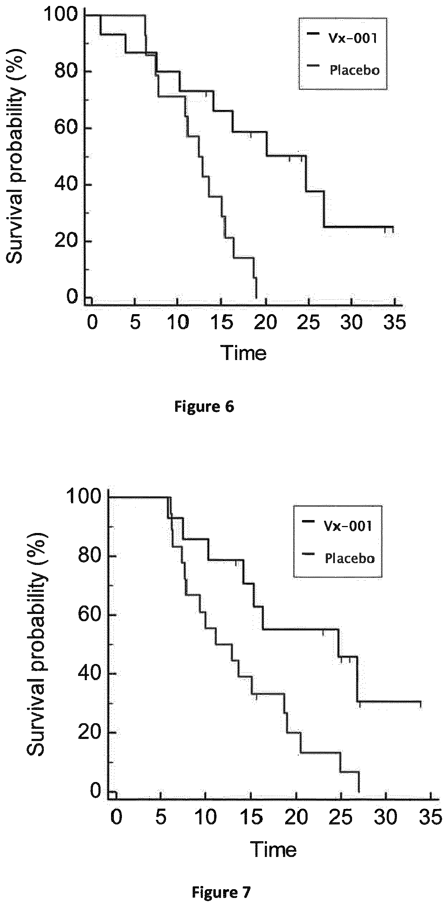

Table 5 shows the clinical outcome of the different sub-groups. Vx-001 significantly prolonged survival in patients without natural immunity (7.9 vs 20.7 mo, p=0.0007, HR=0.29, 95% Cl 0.13-0.67) (Figure 5). In this latter population, there was a significant increase of the 12 mo survival (18.7% vs 82.3% p=0.0004). Vx-001 also significantly prolonged survival in patients who entered the study with stable disease (12.4 vs 24.7 mo, p=0.004, HR=0.33, 95% Cl 0.14-0.78) (Figure 6) and in males (11.1 vs 24.7 mo, p=0.01, HR=0.37, 95% Cl 0.16-0.81) (Figure 7).

**Table 5 Clinical outcome of sub-groups of nonsmoker patients or former smoker that smoke ≤25 years (OS)**

| **Sub-group** | **Median survival (months)** | | **P value** | **HR** | **95% CI** | **12 months OS %** | | **P value** |
|---|---|---|---|---|---|---|---|---|
| | **Placebo** | **Vx-001** | | | | **Placebo** | **Vx-001** | |
| All (n=55) | 11.2 | 16.2 | 0.07 | 0.59 | 0.32-1.06 | 48.3 | 73.1 | 0.09 |

| **Histology** | | | | | | | | |
|---|---|---|---|---|---|---|---|---|
| MH + NSQ (n=42) | 11.1 | 16.2 | 0.06 | 0.53 | 0.26-1.04 | 47.8 | 73.7 | 0.12 |
| SQ (n=13) | 11.2 | 14.1 | 0.84 | 0.89 | 0.28-2.79 | 50 | 71.4 | 0.59 |

| **Response to chemotherapy** | | | | | | | | |
|---|---|---|---|---|---|---|---|---|
| OR (n=26) | 10 | 15.3 | 0.92 | 0.96 | 0.41-2.24 | 40 | 72.7 | 0.13 |
| SD (n=29) | **12.4** | **24.7** | **0.004** | **0.33** | **0.14-0.78** | 57.1 | 73.3 | 0.45 |

| **1st line chemotherapy** | | | | | | | | |
|---|---|---|---|---|---|---|---|---|
| CARBO (n=37) | 10.9 | 15.3 | 0.19 | 0.63 | 0.31-1.27 | 40 | 64.7 | 0.19 |
| CDDP (n=18) | 15.1 | 24.7 | 0.18 | 0.50 | 0.17-1.43 | 66.6 | 88.8 | 0.57 |

| **Natural immunity** | | | | | | | | |
|---|---|---|---|---|---|---|---|---|
| Yes (n=14) | 13.6 | 15.3 | 0.86 | 0.90 | 0.27-2.93 | 75 | 66.6 | 1 |
| No (n=33) | **7.9** | **20.7** | **0.0007** | **0.29** | **0.13-**0.67 | **18.7** | **82.3** | **0.00** 04 |

| **Age** | | | | | | | | |
|---|---|---|---|---|---|---|---|---|
| >65 years (n=31) | 11.2 | 15.3 | 0.27 | 0.64 | 0.29-1.39 | 50 | 69.2 | 0.46 |
| <65 years (n=24) | 10.9 | 20.2 | 0.07 | 0.46 | 0.17-1.19 | 45 | 77 | 0.20 |

| **Sex** | | | | | | | | |
|---|---|---|---|---|---|---|---|---|
| Males (n=32) | **11.1** | **24.7** | **0.01** | **0.37** | **0.16-0.81** | 50 | 78.6 | 0.14 |
| Females (n=23) | 11.2 | 13.3 | 0.78 | 0.89 | 0.37-2.14 | 45.4 | 66.6 | 0.41 |

Regarding the TTF, a significant difference in favor of the Vx-001 was observed in patients without natural immunity (3.1 vs 5.6 mo, p=0.006, HR=0.43 95% Cl 0.20-0.92).

**Table 6: Clinical outcome of sub-groups of nonsmoker patients or former smoker that smoke ≤ 25 years (TTF)**

| **Sub-group** | **Median TTF (months)** | | **P value** | **HR** | **95% CI** |
|---|---|---|---|---|---|
| | Placebo | Vx-001 | | | |
| **Histology** | | | | | |
| MH + NSQ (n=42) | 3.9 | 4.2 | 0.69 | 0.89 | 0.48 - 1.63 |
| SQ (n=13) | 3.0 | 2.9 | 0.69 | 0.81 | 0.27 - 2.46 |

| **Response to chemotherapy** | | | | | |
|---|---|---|---|---|---|
| OR (n=26) | 3.5 | 2.8 | 0.44 | 1.33 | 0.59 - 2.98 |
| SD (n=29) | 3.5 | 3.6 | 0.10 | 0.58 | 0.27 - 1.24 |

| **1st line chemotherapy** | | | | | |
|---|---|---|---|---|---|
| CARBO (n=37) | 3.5 | 2.9 | 0.75 | 0.90 | 0.47 - 1.72 |
| CDDP (n=18) | 3.5 | 5.6 | 0.76 | 0.87 | 034 - 2.21 |

| **Natural immunity** | | | | | |
|---|---|---|---|---|---|
| Yes (n=14) | 4.8 | 2.4 | 0.22 | 1.86 | 0.58 - 5.93 |
| No (n=33) | **3.1** | **5.6** | **0.006** | **0.43** | **0.20 - 0.92** |

| **Age** | | | | | |
|---|---|---|---|---|---|
| >65 years (n=31) | 3.3 | 4.6 | 0.34 | 0.71 | 0.35 - 1.45 |
| <65 years (n=24) | 3.9 | 2.9 | 0.85 | 1.07 | 0.48 - 2.39 |

| **Sex** | | | | | |
|---|---|---|---|---|---|
| Males (n=32) | 3.3 | 2.9 | 0.56 | 0.82 | 0.41 - 1.65 |
| Females (n=23) | 4.8 | 4.2 | 0.96 | 0.98 | 0.43 - 2.22 |

### REFERENCES

Andersen MH and Thor SP. Survivin--a universal tumor antigen. Histol Histopathol. 2002 Apr;17(2):669-75.
Dogan S, Shen R et al. Molecular epidemiology of EGFR and KRAS mutations in 3,026 lung adenocarcinomas: higher susceptibility of women to smoking-related KRAS-mutant cancers. Clin Cancer Res. 2012 Nov 15;18(22):6169-77.
Menez-Jamet J, Gallou C, Rougeot A and Kosmatopoulos K. Optimized tumor cryptic peptides: the basis for universal neo-antigen-like tumor vaccines. Ann Transl Med. 2016 Jul;4(14):266.
Pages F, Kirilovsky A, Mlecnik B, Asslaber M, Tosolini M, Bindea G, Lagorce C, Wind P, Marliot F, Bruneval P, Zatloukal K, Trajanoski Z, Berger A, Fridman WH, Galon J. In situ cytotoxic and memory T cells predict outcome in patients with early-stage colorectal cancer. J Clin Oncol. 2009 Dec 10;27(35):5944-51.
Rizvi NA, Hellmann MD, Snyder A, Kvistborg P, Makarov V, Havel JJ, Lee W, Yuan J, Wong P, Ho TS, Miller ML, Rekhtman N, Moreira AL, Ibrahim F, Bruggeman C, Gasmi B, Zappasodi R, Maeda Y, Sander C, Garon EB, Merghoub T, Wolchok JD, Schumacher TN, Chan TA. Cancer immunology. Mutational landscape determines sensitivity to PD-1 blockade in non-small cell lung cancer. Science. 2015 Apr 3;348(6230):124-8.
Reck et al., Pembrolizumab versus chemotherapy for PDL-1 positive Non-Small-Cell Lung Cancer., N. Engl. J. Med., 2016, 375, 1823-1833
Wang E, Bedognetti D, Marincola FM. Prediction of response to anticancer immunotherapy using gene signatures. J Clin Oncol. 2013 Jul 1;31(19):2369-71.
Zhang et al., The efficacy and safety of anti-PD-1/PD-L1 antibodies for treatment of advanced or refractorycancers: a meta-analysis. Oncotarget, 2016, 7, 73068-73079

## Claims

1. A peptide of SEQ ID No: 2, for use in the treatment of cancer in a HLA-A*0201-positive patient having a non small cell lung cancer (NSCLC) expressing TElomerase Reverse Transcriptase (TERT), wherein said patient either never smoked (never-smoker) or has been a smoker during less than 25 years (light-smoker).

2. A peptide of SEQ ID No: 1, for use in the treatment of cancer in a HLA-A*0201-positive patient having a tumor expressing TERT, wherein said patient is either a never-smoker or a light-smoker.

3. A combination of peptides of SEQ ID No: 1 and SEQ ID No: 2, for use in the treatment of cancer in a HLA-A*0201-positive patient having a tumor expressing TERT, wherein said patient is either a never-smoker or a light-smoker.

4. The peptide or combination of peptides of any of claims 1 to 3, for the use of any of claims 1 to 3, wherein said patient is a never-smoker or a former smoker who has been a smoker during less than 25 years.

5. The peptide or combination of peptides of any of claims 1 to 3, for the use of any of claims 1 to 4, wherein said patient is a non-smoker or a former smoker who stopped smoking at least 5 years before NSCLC diagnosis.

6. The peptide or combination of peptides of any of claims 1 to 3, for the use of any of claims 1 to 5, wherein said patient has received a first line chemotherapy and has a stable disease upon vaccination with the peptide of SEQ ID No: 2.

7. The peptide or combination of peptides of any of claims 1 to 3, for the use of any of claims 1 to 6, wherein said patient has a non-immunogenic tumor.

8. The peptide or combination of peptides of any of claims 1 to 3, for the use of claim 7, wherein the tumor is considered as non-immunogenic if no CTL response specific for the peptide of SEQ ID No: 1 and/or no CTL response specific for the peptide of SEQ ID No: 3 and/or no CTL response specific for the peptide of SEQ ID No: 5 is detectable in a blood sample from the patient before vaccination.

9. The peptide or combination of peptides of any of claims 1 to 3, for the use of claim 8, wherein the blood sample from the patient has been collected less than 2 weeks after the end of a platinum-based first line chemotherapy.

10. The peptide or combination of peptides of any of claims 1 to 3, for the use of any of claims 1 to 10, wherein said patient is a male.

11. A method for *in vitro* determining whether a HLA-A*0201-positive never-smoker or light-smoker patient having a TERT-positive NSCLC is likely to be a good responder to an immunotherapy treatment by vaccination with the peptides of SEQ ID Nos: 1 and 2, comprising assessing the immunogenicity of the tumor, wherein if the tumor is non-immunogenic, the patient is likely to be a good responder to said immunotherapy treatment

12. The method of claim 11, wherein the immunogenicity of the tumor is assessed by measuring CTL response specific for the peptide of SEQ ID No: 1 and/or by measuring CTL response specific for the peptide of SEQ ID No: 3 and/or by measuring CTL response specific for the peptide of SEQ ID No: 5 in a blood sample from said individual before vaccination, wherein if no such CTL response is detectable, the tumor is non-immunogenic.

13. The method of claim 12, wherein the CTL response specific for the peptide of SEQ ID No: 1 and/or the CTL response specific for the peptide of SEQ ID No: 3 and/or the CTL response specific for the peptide of SEQ ID No: 5 is measured by enzyme-linked immunospot (ELISpot) IFNg assay.

14. A kit of parts for performing the method of claim 11 to 13, comprising: (i) reagents and a plate for performing an ELISpot assay, (ii) a peptide selected from the group consisting of SEQ ID No: 1, SEQ ID No: 3 and SEQ ID No: 5 and (iii) an irrelevant peptide as negative control.


SEQUENCE LISTING

<110>  VAXON BIOTECH

<120>  Use of a vaccine targeting a cryptic TERT epitope, for treating
       lung cancer in a HLA-A*0201-positive  never-smoker or
       light-former smoker patient

<130>  B170118EPA-VP9

<140>  EP17305527.8
<141>  2017-05-09

<160>  9

<170>  PatentIn version 3.5

<210>  1
<211>  9
<212>  PRT
<213>  Artificial Sequence

<220>
<223>  peptide from a human protein

<400>  1

Arg Leu Phe Phe Tyr Arg Lys Ser Val
1               5


<210>  2
<211>  9
<212>  PRT
<213>  Artificial Sequence

<220>
<223>  optimized peptide

<400>  2

Tyr Leu Phe Phe Tyr Arg Lys Ser Val
1               5


<210>  3
<211>  9
<212>  PRT
<213>  Artificial Sequence

<220>
<223>  peptide from a human protein

<400>  3

Ile Leu Ala Lys Phe Leu His Trp Leu
1               5


<210>  4
<211>  10
<212>  PRT
<213>  Artificial Sequence

<220>
<223>  peptide from a human protein

<400>  4

Asp Leu Gln Val Asn Ser Leu Gln Thr Val
1               5                   10


<210>  5
<211>  9
<212>  PRT
<213>  Artificial Sequence

<220>
<223>  peptide from a human protein

<400>  5

Leu Thr Leu Gly Glu Phe Leu Lys Leu
1               5


<210>  6
<211>  9
<212>  PRT
<213>  Artificial Sequence

<220>
<223>  peptide from a human protein

<400>  6

Ser Leu Leu Met Trp Ile Thr Gln Val
1               5


<210>  7
<211>  9
<212>  PRT
<213>  Artificial Sequence

<220>
<223>  peptide from a human protein

<400>  7

Thr Leu Glu Glu Ile Thr Gly Tyr Leu
1               5


<210>  8
<211>  9
<212>  PRT
<213>  Artificial Sequence

<220>
<223>  peptide from a human protein

<400>  8

Tyr Leu Glu Tyr Arg Gln Val Pro Asp

1               5



<210>  9
<211>  9
<212>  PRT
<213>  Artificial Sequence

<220>
<223>  peptide from a human protein

<400>  9

Tyr Leu Glu Tyr Arg Gln Val Pro Gly
1               5